# EUROPÄISCHE PATENTSCHRIFT

(11) **EP 0 571 921 B1**
(45) Veröffentlichungstag und Bekanntmachung des Hinweises auf die Patenterteilung: **06.11.1996**
(21) Anmeldenummer: 93108353.9
(22) Anmeldetag: 24.05.1993
(51) Int. Cl.: C07K 7/62, A61K 38/04

(54) **Cyclopeptide und deren Verwendung als Resorptionsförderer bei Applikation auf Schleimhäute**
Cyclopeptides and their use as resorption enhancers in mucous use
Cyclopeptides et leur utilisation pour améliorer la résorption à travers la muqueuse

(30) Priorität: 26.05.1992 DE 4217350
(43) Veröffentlichungstag der Anmeldung: 01.12.1993
(73) Patentinhaber: HOECHST AKTIENGESELLSCHAFT, 65926 Frankfurt am Main (DE)
(72) Erfinder: Sandow, Jürgen, Dr., W-6246 Glashütten (DE); Dürckheimer, Walter, Dr., W-6234 Hattersheim (DE); Ditzinger, Günter, Dr., W-6230 Frankfurt/Main 80 (DE); Merkle, Hans-Peter, Prof., CH-8049 Zürich (CH)

(56) Entgegenhaltungen:
- EP-A- 0 302 466
- DE-A- 2 204 887
- Antimicrob.Agents.Chemother., Vol.24(1), p.:107-113 (1983) (in EP-A-302466 zitiert)

## Beschreibung

Die Erfindung betrifft Cyclopeptide und deren Verwendung zur Förderung der Resorption von Peptiden und Proteinen bei Applikation auf die Schleimhäute.

Die Anwendung von Peptiden und Proteinen als Arzneimittel ist erheblich erschwert durch die Probleme einer geeigneten galenischen Zubereitung, aus der das therapeutisch oder diagnostisch anzuwendende Peptid oder Protein in ausreichender Menge und zuverlässig resorbiert wird.

Bekannt ist die Verabfolgung einer oder mehrerer täglicher Einzeldosen durch nasale Applikation, sei es in Form von Nasentropfen oder durch Einsprühen einer geeigneten Lösung in die Nase [J. Sandow, W. Petri (1985), Transnasal Systemic Medications, Verlag Elsevier, 183 - 199]. Hierzu werden gut verträgliche wäßrige Lösungen mit Zusatz von Konservierungsstoffen und gegebenenfalls von Hilfsstoffen zur Erhöhung der Resorption verwendet. Übliche Hilfsstoffe zur Erhöhung der Resorption (absorption enhancers) sind sämtlich schleimhautreizend oder durch unangenehmen Geruch bzw. Geschmack ungeeignet und führen oft bereits bei einmaliger Applikation zu erheblichem Schmerz und Tränenfluß oder erzeugen bei mehrfacher Applikation eine fortschreitende Reizung und Entzündung der Nasenschleimhäute. Dies gilt z. B. für Derivate der Fusidinsäure, für Gallensäuren, für Tenside und für verschiedene Glykole (Polyethylenglykol, Propylenglykol). In der EP-B-0 302 466 wird ferner die Verwendung von Cyclopeptiden auf Naturstoffbasis zur Förderung der Resorption von Peptiden und Proteinen bei Applikation auf die Schleimhäute beschrieben. Diese Cyclopeptide stellen ein Gemisch chemisch ähnlicher Verbindungen dar; sie müssen daher für eine pharmazeutische Anwendung gereinigt werden. Vor allem ist ihnen eine antibiotische Aktivität eigen, die zur Resistenzentwicklung bestimmter Keime führen kann.

Aufgabe ist es daher, Verbindungen bereitzustellen, die das Wirkungsspektrum von als Arzneimittel eingesetzten Peptiden und Proteinen verbessern, indem sie, bei guter Verträglichkeit und fehlender antibiotischer Partialwirkung, eine gute Resorptionsförderung bewirken.
Diese Aufgabe wird erfindungsgemäß gelöst durch Verbindungen der allgemeinen Formel I 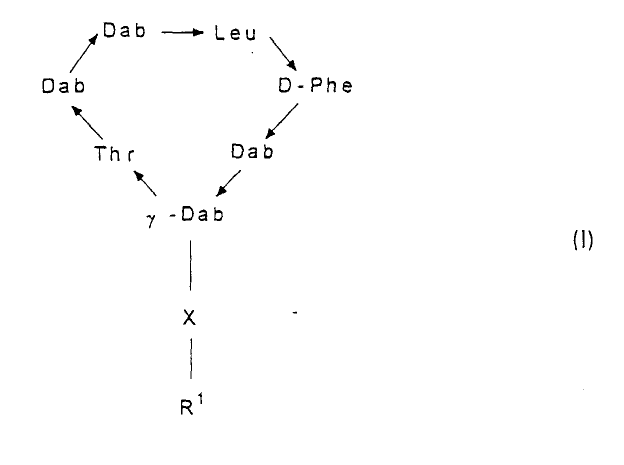 sowie deren physiologisch verträgliche Salze,
worin
- X: Lysin, Ornithin, 2,4-Diaminobuttersäure oder Arginin,
- R¹: einen N-gebundenen Acylrest der Formel II 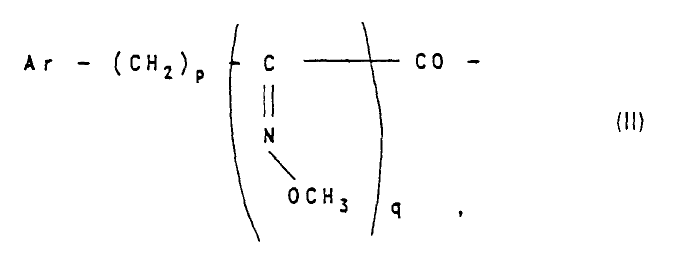
worin Ar für einen Phenylrest, der gegebenenfalls durch 1, 2 oder 3 gleiche oder verschiedene Reste aus der Reihe Hydroxy, (C₁-C₄)-Alkoxy, Amino, Carboxy, (C₁-C₄)-Alkylamino und Halogen substituiert ist, oder
für einen 2-Aminothiazol-4-yl-Rest steht,
- p: eine ganze Zahl von Null bis 4,
- q: Null oder 1 bedeuten,
und die Aminosäuren jeweils in der D- oder L-Form vorliegen können.

Bevorzugt sind solche Reste X, die sich von natürlich vorkommenden Aminosäuren (s. z. B. Schröder, Lübke, The Peptides, Volume 1, New York 1965) und 2,4-Diaminobuttersäure (Dab), deren Antipoden und einfachen Metaboliten ableiten, die, falls chiral, in der D- oder L-Form vorliegen können. γ-Dab steht für 2,4-Diaminobuttersäure, die über die γ-Aminogruppe ringverknüpft ist.

Falls nicht anders angegeben, werden die Dreibuchstabensymbole (vgl. z. B. Pure Appl. Chem. 56 (1984) 595 - 624 und Eur. J. Biochem. 138 (1984) 9 - 37) für die Reste der Aminosäuren verwendet. Diesen Symbolen wird das Symbol 'D' vorangestellt, wenn es sich um den Rest einer D-Aminosäure handelt; Reste ohne Konfigurationssymbol sind L-konfiguriert.

Die Erfindung betrifft sowohl die optisch reinen Verbindungen als auch Stereoisomerengemische wie Enantiomerengemische und Diastereomerengemische.

Bevorzugt sind die folgenden Verbindungen:

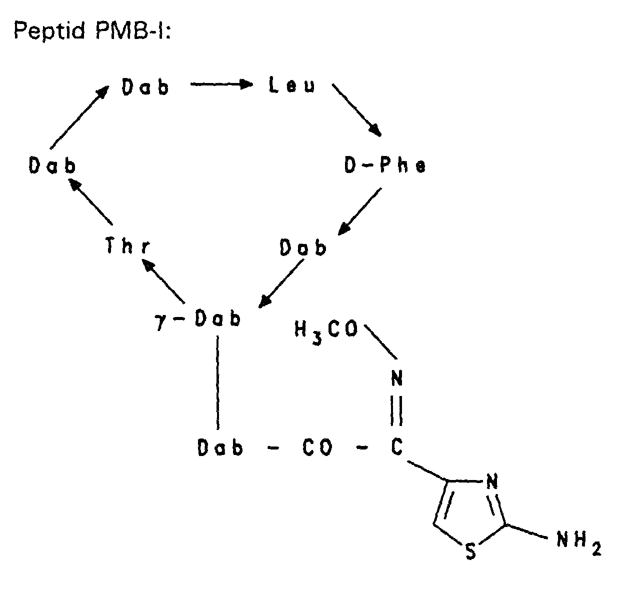

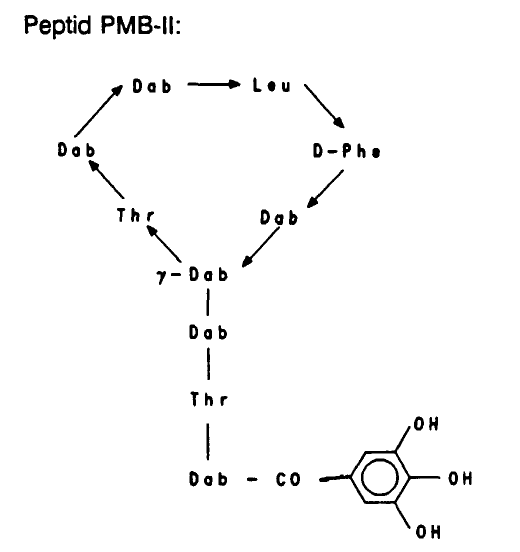

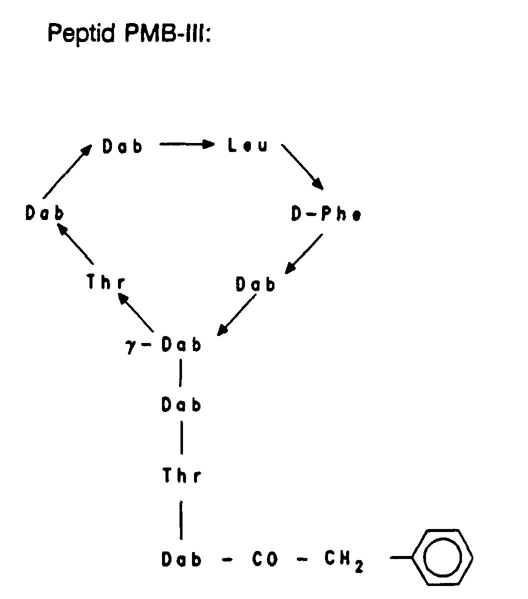

Die erfindungsgemäßen Verbindungen werden hergestellt z.B. unter Benutzung der allgemeinen Methoden der Peptidchemie (Houben-Weyl, Methoden der organischen Chemie, 15/1 und 2). Die Verbindungen können hergestellt werden, beispielsweise stufenweise ausgehend von Polymyxin B-Oktapeptid (Formel I; X = L-Dab; R¹ = H) deren Aminogruppen mit Ausnahme der zu acylierenden geschützt sind und den entsprechenden aktivierten Carbonsäuren. (z. B. Carbonsäurehalogenide, gemischte Anhydride oder Aktivester.)

Die Ausgangspeptide können durch reduktive Spaltung von N-BOC-geschütztem Polymyxin B beispielsweise mit Metallhydriden, z. B. NaBH₄, NaCNBH₃, oder durch enzymatische Spaltung, z. B. mit der literaturbekannten Protease Ficin, erhalten werden. Als N-Schutzgruppe hat sich in diesem Fall die N-Oxido-pyridyl-methyloxycarbonyl-Schutzgruppe bewährt.

Ein weiterer Gegenstand der Erfindung ist die Verwendung von Verbindungen der Formel I zur Förderung der Resorption von Peptiden und Proteinen bei Applikation auf die Schleimhäute. Die erfindungsgemäßen Verbindungen tragen in ganz erheblichem Maße zur Verbesserung der Resorption von Peptiden und Proteinen bei Applikation auf die Schleimhäute bei. So beträgt die Steigerung der Wirksamkeit (beruhend auf einer verbesserten Resorption) der Peptide oder Proteine nach dem Zusatz der erfindungsgemäßen Verbindungen 300 bis 400 % und kann im Einzelfall mehr als 1000 % betragen.

Darüber hinaus verursachen die erfindungsgemäßen Verbindungen beim Menschen keine Schmerzempfindung und Schleimhautschädigung bei nasaler Applikation von 1 bis 200 µl in einer Konzentration von 10⁻⁵ bis 10⁻¹ mol/l. Die lokale Anwendung der gleichen Konzentration durch vaginale, rektale oder buccale Arzneiformen (d. h. z. B. Filme, Tabletten, Suppositorien) führt ebenfalls zu keiner Schleimhautreizung.

Für die meisten der heute bekannten Peptide und Proteine, die als Therapeutika oder Diagnostika verwendet werden oder in nächster Zeit zur Anwendung kommen werden, ist eine Applikation auf die Schleimhäute, wie z.B. die nasale, buccale, rektale, vaginale oder pulmonale Anwendung, insbesondere jedoch die nasale, zweckvoll und möglich.

Hierfür eignen sich Peptide und Proteine, die aus 3 bis 225 Aminosäuren bestehen, wie z. B. TRH (Protirelin, Thyroliberin), LHRH (Gonadoliberin), chemisch modifizierte Analogpeptide der hypothalamischen regulatorischen Hormone wie z.B. Buserelin, Somatostatin und cyclische Somatostatinanaloga, Somatorelin, (GRH)-Analoga, Analogpeptide von Hypophysenhormonen wie z. B. das Corticotropin-Analogon Alsactid (ACTH-17), Calciumregulierende Hormone (Calcitonin, Parathormon) und ihre Analoga sowie gastrointestinale Hormone (z. B. Secretin und Cholecystokinin) und pankreatische Hormone (Insulin und Insulin-Analoga). Besonders geeignet sind solche mit 3 bis 51 Aminosäuren.

Insbesondere seien genannt:

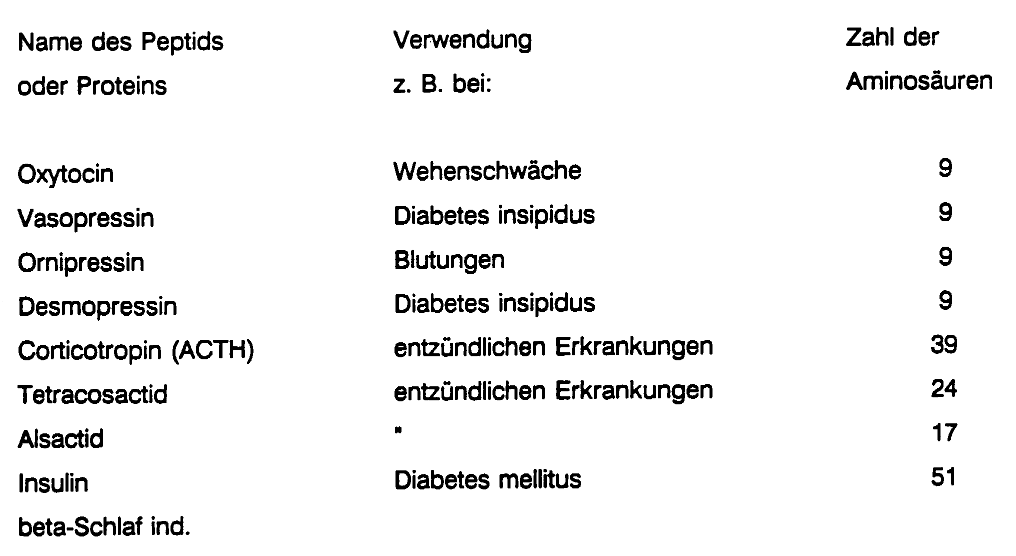

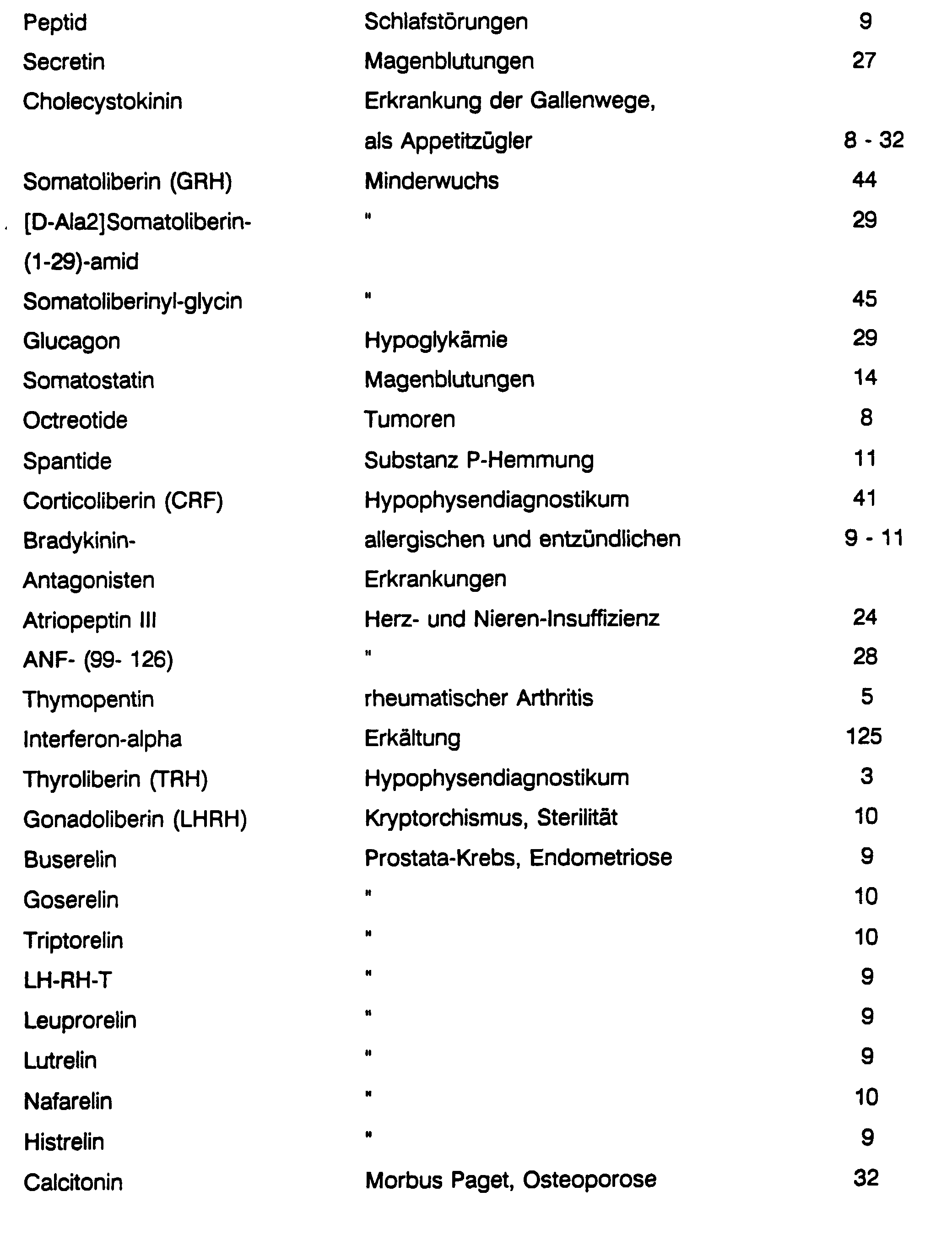

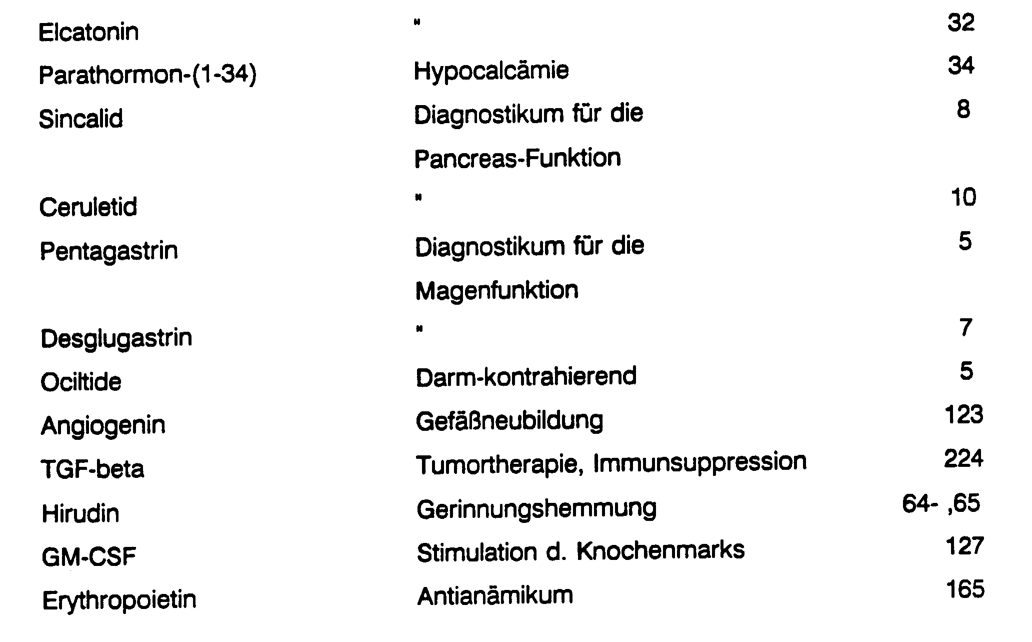

Diese Peptide und Proteine können nach allgemein bekannten Verfahren, z.B. durch Merrifield-Synthese, Genetic Engineering und durch Isolierung natürlich vorkommender Peptide und Proteine, erhalten werden.

Die Erfindung betrifft ferner pharmazeutische Zubereitungen, enthaltend
a) eine pharmakologisch wirksame Menge eines, zweier oder dreier Peptide oder Proteine, jeweils enthaltend 3 bis 225 Aminosäuren, insbesondere 3 bis 51 Aminosäuren, oder deren physiologisch verträgliche Salze und
b) mindestens einen Hilfsstoff der allgemeinen Formel I.

Bevorzugt sind solche Zubereitungen, die mindestens einen Hilfsstoff, ausgewählt aus den Peptiden PMB-I bis -III, enthalten.

Die erfindungsgemäßen pharmazeutischen Zubereitungen enthalten ferner bevorzugt ein Peptid oder Protein enthaltend 3 bis 225 Aminosäuren, insbesondere ein Peptid oder Protein enthaltend 3 bis 51 Aminosäuren.

Jedoch auch Zubereitungen, enthaltend zwei oder drei verschiedene Peptide und/oder Proteine, wie beispielsweise Corticotropin + LHRH + GRH oder Protirelin + LHRH + GRH, in Verbindung mit mindestens einem Hilfsstoff der Formel I, sind von Interesse, insbesondere zur Anwendung als Diagnostikum.

Die Dosis der Peptide und/oder Proteine und der Hilfsstoffe der Formel I bei der Anwendung bei Säugern, vorzugsweise beim Menschen, liegen in den erfindungsgemäßen Zubereitungen bzw. Erzeugnissen im Bereich von 10 µg bis 10 mg pro Peptid/ Protein und Anwendung und für den Hilfsstoff der Formel I bei einer Konzentration von 10⁻⁵ bis 10⁻¹ mol/l pro Anwendung, bevorzugt zwischen 10⁻⁴ und 10⁻² mol/l.

Die Anwendung der erfindungsgemäßen Zubereitungen kann durch Applikation auf die Schleimhäute, d. h. nasal, buccal, rektal, pulmonal oder vaginal erfolgen. Die nasale Applikation ist hierbei bevorzugt.

Die erfindungsgemäßen pharmazeutischen Zubereitungen können nach dem Fachmann bekannten Verfahren unter Hinzufügung von zur Herstellung von pharmazeutischen Präparaten geeigneten Trägerstoffen hergestellt werden. Es eignen sich insbesondere Arzneiformen zur Applikation auf die Schleimhäute, wie beispielsweise Tabletten, Suppositorien, Kapseln, Gele, Filme, Emulsionen, Suspensionen, Aerosole, Lösungen oder Sprays (Sucker, Fuchs, Speiser, Pharmazeutische Technologie, Georg Thieme Verlag 1978).

Vorzugsweise verwendet werden:

### 1. Wäßrige oder wäßrig-alkoholische Lösungen zur Applikation mit einer Tropfpipette bzw. einer Kunststoffquetschflasche oder zur Vernebelung mit einer Dosierzerstäuberpumpe

Die Zubereitung kann neben dem Wirkstoff und dem Resorptionsförderer, z.B. einen isotonisierenden Zusatz, z. B. Natriumchlorid, Kaliumnitrat, Kalium-Natrium-Phosphat, Polyalkohole, wie z. B. Glucose, Mannit, Sorbit, Puffersubstanzen, wie beispielsweise Kalium-Natrium-Phosphat, Citronensäure und ihre Salze sowie Mischungen der beiden, um einen pH-Bereich von 3 bis 8 einzustellen, ein Konservierungsmittel, z. B. Benzalkoniumchlorid, Benzylalkohol, 1,1,1-Trichlor-2-methyl-2-propanol, Methyl-4-hydroxybenzoat, einen Chelatbildner, z.B. Natrium-EDTA, und als Lösungsmittel Wasser oder Mischungen von Wasser mit (C₁-C₄)-Alkanolen enthalten. Die Lösung wird mit einem geeigneten Gerät appliziert bzw. in die Nase oder auf die Mundschleimhaut gesprüht.

### 2. Wäßrige oder wäßrig-alkoholische Gele zum Einbringen in Körperhöhlen (Mund, Nase, Rektum, Vagina)

Zusätzlich zu 1. enthält ein Gel einen die Viskosität erhöhenden Zusatz, z. B. ein Polyacrylatpolymer oder einen Celluloseether wie z. B. Hydroxypropylmethylcellulose (HPMC), Hydroxyethylcellulose (HEC), Methylhydroxyethylcellulose (MHEC).

### 3. Suspensionen in Treibgasen

Die Zubereitung kann neben dem mikronisierten Wirkstoff und dem mikronisierten Resorptionsförderer als Treibgas einen Fluorchlorkohlenwasserstoff, z. B. ®Frigen F 11, 12 oder 114 (® = ein eingetragenes Warenzeichen der Hoechst AG), bzw. deren Gemische oder einen Fluorkohlenwasserstoff wie Tetrafluorethan (HFA 134 a) oder Heplafluorpropan (HFA 227) und ein Suspendierhilfsmittel, z.B. Sorbitantrioleat, Ölsäure oder Lecithin enthalten. Die Abfüllung erfolgt in an sich bekannter Weise nach dem Kälteabfüllverfahren oder aber durch Druckfüllung.

### 4. Verreibungen mit Trägerhilfsstoffen abgefüllt in Kapseln zur intranasalen bzw. inhalativen Anwendung

Die mikronisierten Stoffe (Wirkstoff und Resorptionsförderer) werden, gegebenenfalls nach Zugabe eines Mittels zur Verbesserung der Fließeigenschaften, wie Lactose, in Hartgelatinekapseln abgefüllt. Der Inhalt einer Kapsel wird mit einer Inhalationshilfe, die es erlaubt, das Pulver in einen inhalierbaren Rauch zu überführen, intranasal bzw. pulmonal appliziert.

### 5. Buccalformen

Wirkstoff und Resorptionsförderer können in gelöster oder suspendierter Form vorliegen. Als Arzneiformen eignen sich Komprimate oder Laminate aus Mischungen von Wirkstoff und Absorptionsförderer in Polymeren. Als Polymere kommen Celluloseether (z. B. HPMC, Carboxymethylcellulose (CMC)) oder Polyacrylate in Frage.

### Beispiel 1: Synthese von PMB-I

Man löst 1.26 g (1 mmol) "Tetra-Boc-Polymyxin B-Oktapeptid" in 10 ml Pyridin und fügt 0.318 g HOBt-Ester (HOBt = 1-Hydroxybenzotriazol) der 2-Amino-thiazol-4-yl-2-methoximinoessigsäure hinzu. Nach 24 Stunden dampft man das Pyridin im Vakuum ab, löst den Rückstand in 200 ml Essigsäureethylester und wäscht die Lösung zunächst mit 200 ml n/10 Salzsäure und danach mit 200 ml Wasser. Nach dem Trocknen über Natriumsulfat entfernt man das Lösungsmittel, löst erneut in einem Gemisch Essigsäureethylester/Methanol 85 : 15 und chromatographiert mit dem gleichen Lösungsmittelgemisch an einer Kieselgel-Säule (4 x 50 cm; 70 - 200 µm; 200 g). 40 Fraktionen à 10 ml werden aufgefangen. Die Fraktionen 21 - 35 enthalten den gewünschten Stoff. Das Lösungsmittel wird im Vakuum am Rotationsverdampfer entfernt.
Ausbeute des Zwischenprodukts: 1.1 g (76 % d. Th)
Molmasse: 1445 g/mol (C₆₅H₁₀₄N₁₆O₁₉S)
R_{f}-Wert [DC-Kieselgel 60 F 254/Merck, Darmstadt, Essigsäurester/Methanol 85 : 15] = 0.51

Abspaltung der Boc-Schutzgruppen:
Man löst 1.1 g des Zwischenprodukts in 10 ml Trifluoressigsäure unter Eiskühlung und läßt dann 2 Stunden bei Raumtemperatur stehen. Nach Einengen verreibt man den festen Rückstand mit Äther, filtriert, wäscht mit Äther nach und trocknet über P₂O₅ im Vakuum.
Ausbeute: 0.96 g (78.2 %)
Molmasse: 1046 g/mol (C₄₅H₇₂N₁₆O₁₁S x 5 CF₃CO₂H)
R_{f}-Wert [DC-Kieselgel 60 F 254/Merck, Darmstadt, Laufmittel n-Butanol (50), Pyridin (20), Eisessig (6), Wasser (24)] = 0.11.

### Beispiel 2: Synthese von PMB-II

Man löst 1.26 g (1 mmol) "Tetra-Boc-Polymyxin B-Oktapeptid" in 10 ml Pyridin und fügt 0.287 g HOBt-Ester der Gallussäure hinzu. Nach 48 Stunden zieht man das Pyridin ab, löst den Rückstand in 200 ml Essigsäureethylester und wäscht diese Lösung zunächst mit 200 ml n/10 Salzsäure und danach mit 200 ml Wasser. Nach dem Trocknen über Natriumsulfat entfernt man das Lösungsmittel, löst erneut in einem Gemisch von Essigsäureethylester/Methanol (85 : 15) und chromatographiert mit dem gleichen Lösungsmittelgemisch. Als Trägermaterial benutzt man Kieselgel (70 - 200 µm), das vorher zur Entfernung von Eisen mit halbkonzentrierter Salzsäure und anschließend bis zur neutralen Reaktion mit Wasser gewaschen und bei 130 °C getrocknet wurde. 40 Fraktionen à 15 ml werden eluiert. Die Fraktionen 34 - 39 enthalten den gewünschten Stoff. Das Lösungsmittel wird im Vakuum am Rotationsverdampfer entfernt.
Ausbeute des Zwischenprodukts: 0.89 g (62.8 %)
Molmasse = 1414.58 g/mol.

Die Abspaltung der Boc-Schutzgruppen wurde analog Beispiel 1 durchgeführt.
Ausbeute: 0.79 g (86 %)
Molmasse: 1014 g/mol (C₄₆H₇₁N₁₃O₁₃ x 4CF₃CO₂H = 1470.21)
R_{f}-Wert (Trennsystem wie unter Beispiel 1): 0.05.

### Beispiel 3: Synthese von PMB-III

Man löst 1.26 g (1 mmol) "Tetra-Boc-Polymyxin B-Oktapeptid" in 10 ml Pyridin und fügt 0.233 g (1 mmol) Hydroxysuccinimidester der Phenylessigsäure zu. Nach 20 Stunden Reaktionszeit bei Raumtemperatur löst man den Rückstand in 200 ml Essigsäureethylester, wäscht ihn mit 200 ml n/10 Salzsäure und mit 200 ml Wasser. Nach dem Trocknen über Natriumsulfat entfernt man das Lösungsmittel und löst erneut in einem Gemisch Essigsäureethylester/Methanol (85 : 15) und chromatographiert an einer Kieselgelsäule (4 x 50 cm; 70 - 200 µm; 200 g) mit dem gleichen Lösungsmittelgemisch. Es werden 40 Fraktionen á 15 ml abgenommen. Die Fraktionen 20-39 enthalten das gewünschte Produkt (C₆₇H₁₀₅N₁₃O₁₈). Das Lösungsmittel wird im Vakuum am Rotationsverdampfer entfernt.
Ausbeute des Zwischenprodukts: 0.85 g (61.2 %)
Molmasse: 1386 g/mol (Li-Salz)
R_{f}-Wert (DC wie Beispiel 1) = 0.57

Die Abspaltung der Schutzgruppen wurde wie in Beispiel 1 durchgeführt.
Ausbeute: 0.59 g (97.7 %)
Molmasse: 980 g/mol (C₄₇H₇₃N₁₃O₁₀).
R_{f}-Wert (DC-Lösungsmittelgemisch wie in Beispiel 1): 0.26

### Beispiel 4: Untersuchung der Wirkungsverstärkung von LHRH oder LHRH-Analogen

Eine zum Nachweis der Wirkungsverstärkung eines Peptids oder Proteins mittels eines erfindungsgemäßen Cyclopeptids geeignete Methode ist z. B. die Bestimmung der LH-Freisetzung bei männlichen, durch Ethylcarbamat narkotisierten Ratten (100 g Körpergewicht). Dabei wird die Hormonfreisetzung über einen Zeitraum von 6 - 7 Stunden nach Behandlung, das heißt nach nasaler oder rektaler Applikation von LHRH oder LHRH-Analogen in physiologischer Kochsalzlösung mit oder ohne Zusatz von Cyclopeptiden der Formel I, verglichen.

Es zeigte sich, daß z. B. durch die Peptide PMB-I bis -III in einer Konzentration von 0.01 M eine 21 - 32fache Wirkungssteigerung einer nasal verabreichten Dosis von 10 ng Buserelin erzielt wird, bezogen auf die LH-Freisetzung. Die Wirkung einer nasal verabreichten Dosis von 800 ng LHRH wurde durch die Peptide PMB-I - III im gleichen Tiermodell um den Faktor 3 bis 6 verstärkt, ebenfalls bezogen auf die Flächen unter den Kurven.

### Beispiel 5: Untersuchung der Wirkungsverstärkung von ACTH

Die Wirkung von Cyclopeptiden der Formel I auf die Resorption von ACTH (Corticotropin) und ACTH-Analogen wurde an männlichen durch Pentobarbital oder Ether narkotisierten Ratten (100 g Körpergewicht) nach Applikation der Testsubstanzen gemäß Beispiel 4 untersucht. Als Parameter der Wirkung wurde die Corticosteron-Freisetzung im Serum durch spezifischen Radioimmunoassay bestimmt. Es zeigte sich, daß nach nasaler Behandlung z. B. mit dem ACTH-Analogen Alsactid (ACTH-17) in einer Dosis von 1.5 µg in Gegenwart der Peptide PMB-I bis III die Corticosteron-Freisetzung über 3 h um das 2 - 3fache gesteigert wurde.

### Beispiel 6: Untersuchung der Wirkungsverstärkung von Calcitonin

Die Wirkung von Cyclopeptiden der Formel I auf die Resorption von Calcitonin und Calcitonin-Analogen (z. B Lachs-Calcitonin) wurde an männlichen Ratten von 100 oder 200 g Körpergewicht, nach Applikation gemäß Beispiel 4, durch Bestimmung der Serum-Calciumspiegel über einen Zeitraum von 1 - 6 h nach der Behandlung untersucht.
Es wurde gefunden, daß die Wirkung einer nasalen Gabe von 1.2 µg Lachs-Calcitonin durch den Zusatz z. B. von Peptid PMB-I in einer Konzentration von 0.01 M um das 3.4fache gesteigert wurde, bezogen auf den Rückgang des Serum-Calciumspiegels.

### Beispiel 7: Untersuchung der Wirkungsverstärkung von Insulin

Die Wirkung von Cyclopeptiden der Formel I auf die nasale Resorption von Humaninsulin oder anderem Insulin wurde an männlichen durch Ether narkotisierten Ratten mit einem Körpergewicht von 100 g nach Applikation der Testsubstanzen gemäß Beispiel 4 untersucht. Als Parameter für die Wirkung dient der Rückgang des Blut-Glucosespiegels.
Dabei zeigte sich, daß die Peptide PMB-I bis III in einer Konzentration von 0.01M die nasale Resorption von 20 IE/kg Humaninsulin um den Faktor 3 - 4 verbessern konnten.

### Beispiel 8: Untersuchung der Schleimhautverträglichkeit

Die Verträglichkeit von Cyclopeptiden der Formel I, welche als Hilfsstoffe für die Erhöhung der Resorption verwendet werden, kann an der isolierten Magenschleimhaut des Meerschweinchens geprüft werden. [Wirth K, Bickel M & Deutschländer N (1987): Patent blue permeation through the isolated guinea pig gastric mucosa: a quantitative method for the assessment of gastric irritants. Med. Sci. Res. 15, 881-882]. In einem ähnlichen Modell wird die Nasalschleimhaut des Rindes verwendet. [Ditzinger G, Sandow J, Merkle HP (1990): In vitro model for nasal peptide delivery: Enhancement effects of cyclopeptides on the transport rate of two oligopeptides. Proceed. Intern. Symp. Contr. Rel. Bioact. Mater. 17, 220-221]. Mit dem letzteren Modell wurde die Permeation von Patentblau als Markersubstanz für eine Schleimhautschädigung untersucht. Während als Resorptionsverbesserer verwendete Gallensäuren wie Natriumdesoxycholat oder Natriumglycocholat oder als Resorptionsverbesserer verwendete Tenside wie Polidocanol (Laureth-9) die Permeation von Patentblau um ca. 300 % erhöhten, zeigten die Peptide PMB-I bis III keinen Einfluß auf die Permeationsrate.

### Beispiel 9: Nasallösung

| | |
|---|---|
| Buserelin | 0,15 mg |
| Peptid PMB-I | 1,50 mg |
| Natriumchlorid | 0,80 mg |
| Citronensäure • H₂O | 0,11 mg |
| Natriumcitrat • 2H₂O | 0,15 mg |
| Benzalkoniumchlorid | 0,01 mg |
| Dinatrium-EDTA | 0,01 mg |
| Wasser (gereinigt) ad | 0,1000 ml |

### Beispiel 10: Gel

| | |
|---|---|
| Alsactid (ACTH-17) | 0,003 mg |
| Peptid PMB-II | 1,200 mg |
| Polyacrylsäure 940 | 0,400 mg |
| Natriumhydroxid Lösung 15 % | 0,900 mg |
| Glycerin | 15,000 mg |
| Methyl-4-hydroxybenzoat | 0,150 mg |
| Gereinigtes Wasser | ad 100,000 mg |

### Beispiel 11: Suppositorium

| | |
|---|---|
| Lachs-Calcitonin | 0,200 mg |
| Peptid PMB-III | 1,000 mg |
| Suppositorienmasse (Hartfett) | ad 2,500 g |

### Beispiel 12: Diagnostikum

| | |
|---|---|
| Protirelin | 0,050 mg |
| Gonadoliberin | 0,025 mg |
| Somatoliberin | 0,025 mg |
| Peptid PMB-I | 0,250 mg |
| Citronensäure • H₂O | 0,170 mg |
| Dinatriummonohydrogenphosphat • 12H₂O | 1,100 mg |
| Natriumchlorid | 0,600 mg |
| Benzylalkohol | 1,000 mg |
| Gereinigtes Wasser | ad 0,100 ml |

## Patentansprüche

1. Verbindung der allgemeinen Formel I 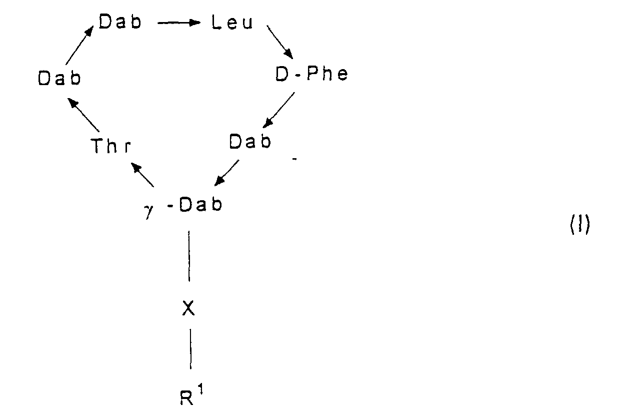 sowie deren physiologisch verträgliche Salze,
worin
X Lysin, Ornithin, 2,4-Diaminobuttersäure oder Arginin,
R¹ einen N-gebundenen Acylrest der Formel II 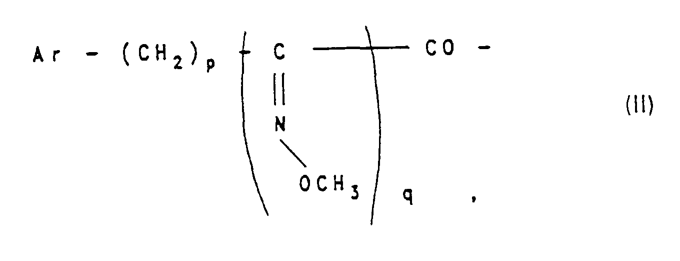
worin Ar für einen Phenylrest, der gegebenenfalls durch 1, 2 oder 3 gleiche oder verschiedene Reste aus der Reihe Hydroxy, (C₁-C₄)-Alkoxy, Amino, Carboxy, (C₁-C₄)-Alkylamino und Halogen substituiert ist, oder
für einen 2-Aminothiazol-4-yl-Rest steht,
p eine ganze Zahl von Null bis 4,
q Null oder 1 bedeuten,
und die Aminosäuren jeweils in der D- oder L-Form vorliegen können.

2. Verbindung der Formel 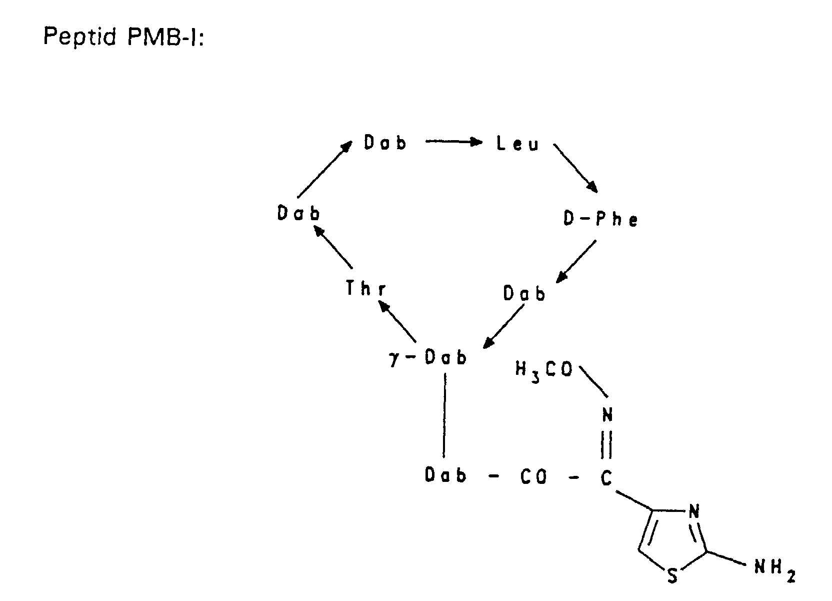 oder deren physiologisch verträgliche Salze.

3. Verbindung der Formel 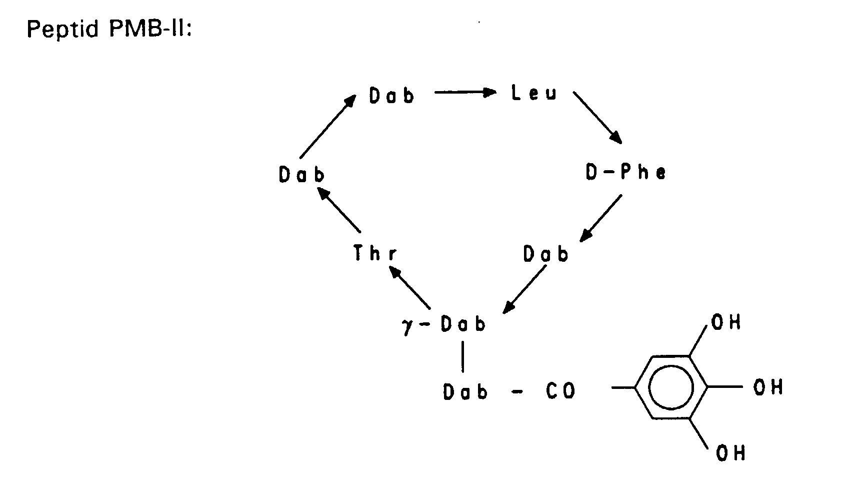 oder deren physiologisch verträgliche Salze.

4. Verbindung der Formel 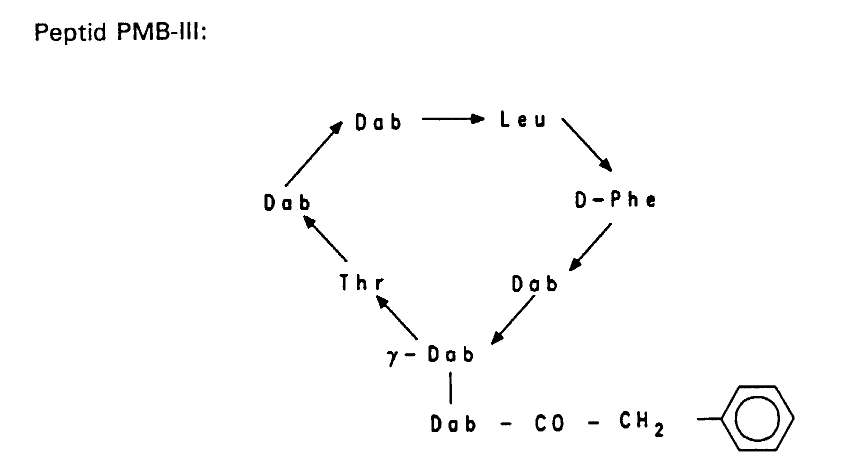 oder deren physiologisch verträgliche Salze.

5. Verwendung mindestens einer Verbindung gemäß einem oder mehrerer der Ansprüche 1 bis 4 zur Herstellung einer pharmazeutischen Zubereitung zur Föderung der Resorption von Peptiden und Proteinen bei Applikation auf die Schleimhaut.

6. Pharmazeutische Zubereitung, enthaltend
a) eine pharmakologisch wirksame Menge eines, zweier oder dreier Peptide oder Proteine, jeweils enthaltend 3 bis 225 Aminosäuren, insbesondere 3 bis 51 Aminosäuren, oder deren physiologisch verträgliche Salze und
b) mindestens einen Hilfsstoff der allgemeinen Formel I 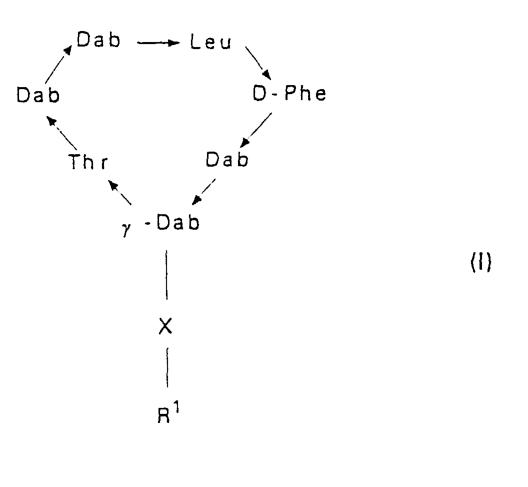
oder ein physiologisch verträgliches Salz dieses Hilfstoffs,
wobei in Formel I
X Lysin, Ornithin, 2,4-Diaminobuttersäure oder Arginin,
R¹ einen N-gebundenen Acylrest der Formel II 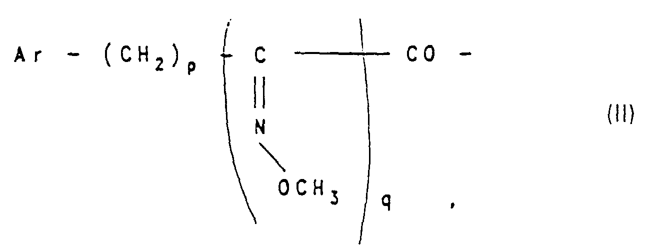
worin Ar für einen Phenylrest, der gegebenenfalls durch 1, 2 oder 3 gleiche oder verschiedene Reste aus der Reihe Hydroxy, (C₁-C₄)-Alkoxy, Amino, Carboxy, (C₁-C₄)-Alkylamino und Halogen substituiert ist, oder
für einen 2-Aminothiazol-4-yl-Rest steht,
p eine ganze Zahl von Null bis 4,
q Null oder 1, bedeuten,
und die Aminosäuren jeweils in der D- oder L-Form vorliegen können.

7. Pharmazeutische Zubereitung gemäß Anspruch 6, dadurch gekennzeichnet, daß sie ein Peptid oder Protein, enthaltend 3 bis 225 Aminosäuren, enthält.

8. Pharmazeutische Zubereitung gemäß einem oder mehrerer der Ansprüche 6 und 7, dadurch gekennzeichnet, daß sie ein Peptid oder Protein, enthaltend 3 bis 51 Aminosäuren, enthält.

9. Verfahren zur Herstellung einer Zubereitung gemäß einem oder mehrerer der Ansprüche 6 bis 7, dadurch gekennzeichnet, daß man
a) ein, zwei oder drei Peptide oder Proteine, jeweils enthaltend 3 bis 225 Aminosäuren, insbesondere
3 bis 51 Aminosäuren, oder deren physiologisch verträgliche Salze und
b) mindestens einen Hilfsstoff der allgemeinen Formel I
in eine für die Applikation auf die Schleimhaut geeignete Darreichungsform bringt.

## Claims

1. A compound of the formula I 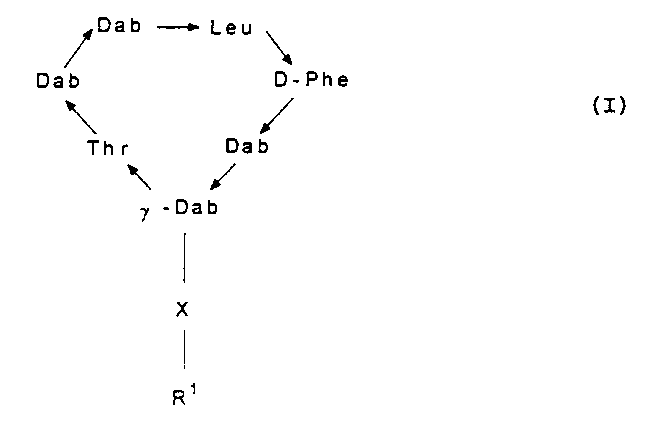 or a physiologically tolerated salt thereof,
in which
X is lysine, ornithine, 2,4-diaminobutyric acid or arginine,
R¹ is an N-bonded acyl radical of the formula II 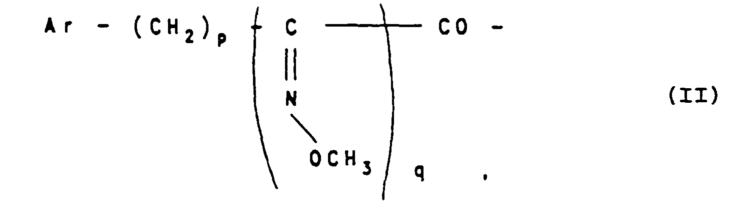
in which Ar is a phenyl radical, which is optionally substituted by 1, 2 or 3 identical or different radicals from the series comprising hydroxyl, (C₁-C₄)-alkoxy, amino, carboxyl, (C₁-C₄)-alkylamino and halogen, or
is a 2-aminothiazol-4-yl radical,
p is an integer from zero to 4,
q is zero or 1,
and the amino acids may each be present in the D- or L-form.

2. The compound of the formula 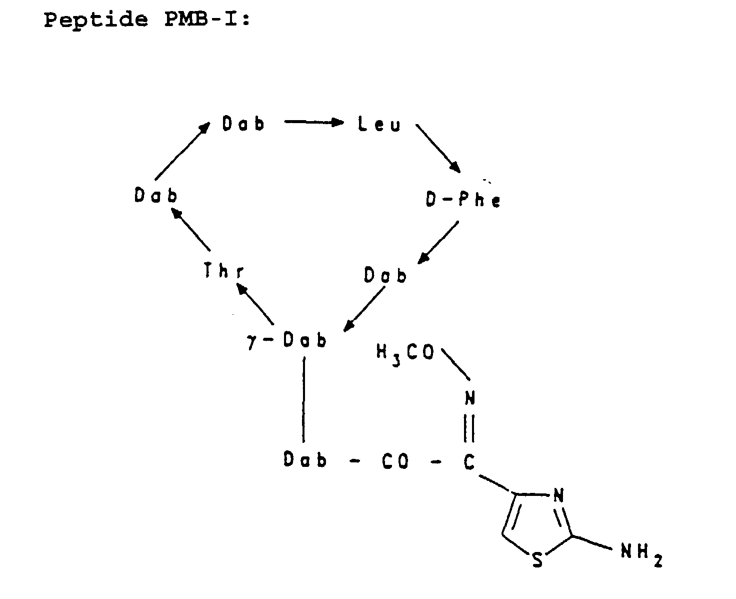 or a physiologically tolerated salt thereof.

3. The compound of the formula 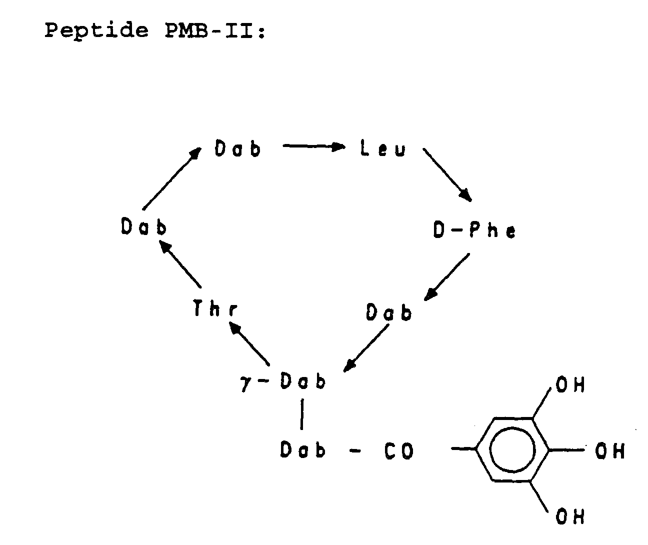 or a physiologically tolerated salt thereof.

4. The compound of the formula 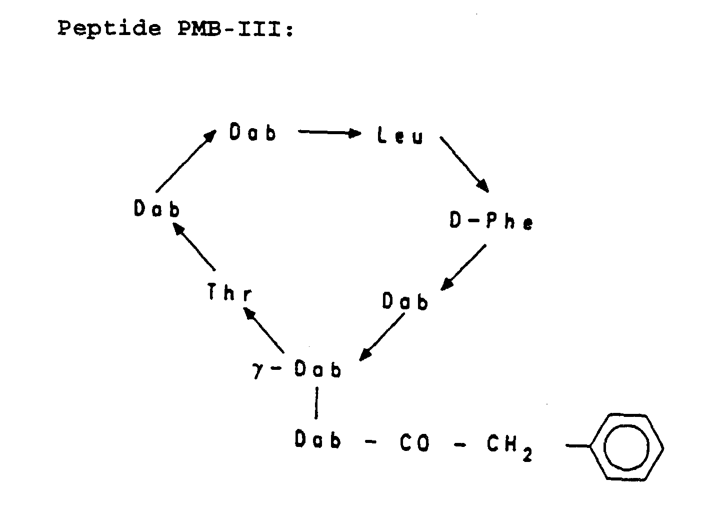 or a physiologically tolerated salt thereof.

5. The use of at least one compound as claimed in one or more of claims 1 to 4 for the preparation of a pharmaceutical formulation for promoting the absorption of peptides and proteins when applied to the mucosa.

6. A pharmaceutical formulation comprising
a) a pharmacologically active amount of one, two or three peptides or proteins in each case containing 3 to 225 amino acids, in particular 3 to 51 amino acids, or physiologically tolerated salts thereof, and
b) at least one auxiliary of the formula I 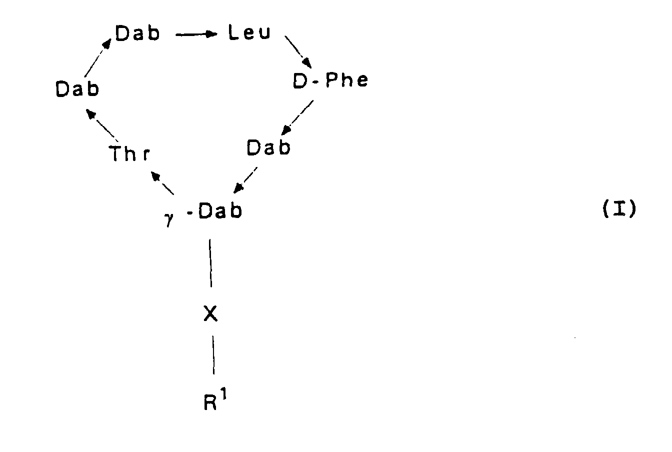 or a physiologically tolerated salt of this auxiliary,
where in formula I
X is lysine, ornithine, 2,4-diaminobutyric acid or arginine,
R¹ is an N-bonded acyl radical of the formula II 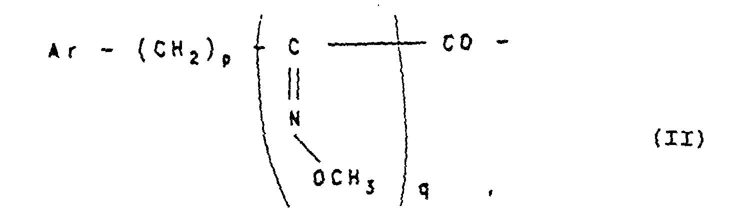
in which Ar is a phenyl radical, which is optionally substituted by 1, 2 or 3 identical or different radicals from the series comprising hydroxyl, (C₁-C₄)-alkoxy, amino, carboxyl, (C₁-C₄)-alkylamino and halogen, or
is a 2-aminothiazol-4-yl radical,
p is an integer from zero to 4,
q is zero or 1,
and the amino acids may each be present in the D- or L-form.

7. A pharmaceutical formulation as claimed in claim 6, which comprises a peptide or protein containing 3 to 225 amino acids.

8. A pharmaceutical formulation as claimed in one or more of claims 6 and 7, which comprises a peptide or protein containing 3 to 51 amino acids.

9. A process for the preparation of a formulation as claimed in one or more of claims 6 to 7, which comprises bringing
a) one, two or three peptides or proteins in each case containing 3 to 225 amino acids, in particular 3 to 51 amino acids, or physiologically tolerated salts thereof, and
b) at least one auxiliary of the formula I
into a suitable administration form for application to the mucosa.

## Revendications

1. Composés de Formule générale I 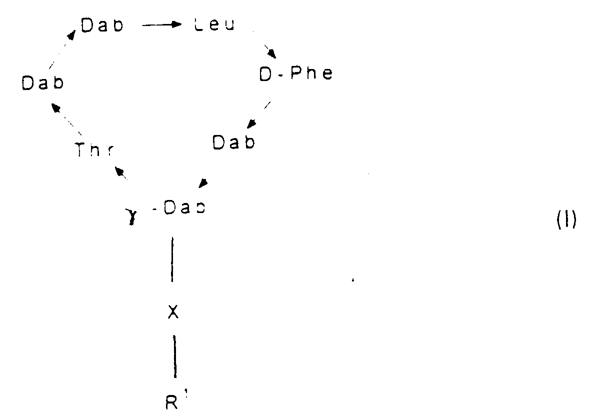 ainsi que leurs sels physiologiquement compatibles,
dans laquelle
X représente la lysine, l'ornithine, l'acide 2,4-diaminobutyrique ou l'arginine,
R¹ représente un radical acyle de Formule II lié à un azote 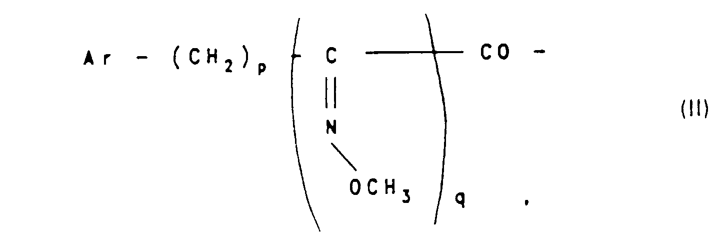
formule dans laquelle Ar représente un radical phényle éventuellement substitué par 1, 2 ou 3 groupes identiques ou différents choisis parmi les groupes hydroxy, alcoxy en C₁-C₄, amino, carboxy, alkylamino en C₁-C₄ et les atomes d'halogène, ou
un radical 2-aminothiazol-4-yle,
p signifie un nombre entier compris entre zéro et 4,
q zéro ou 1,
et les aminoacides peuvent être respectivement sous la forme D ou L.

2. Composé de Formule 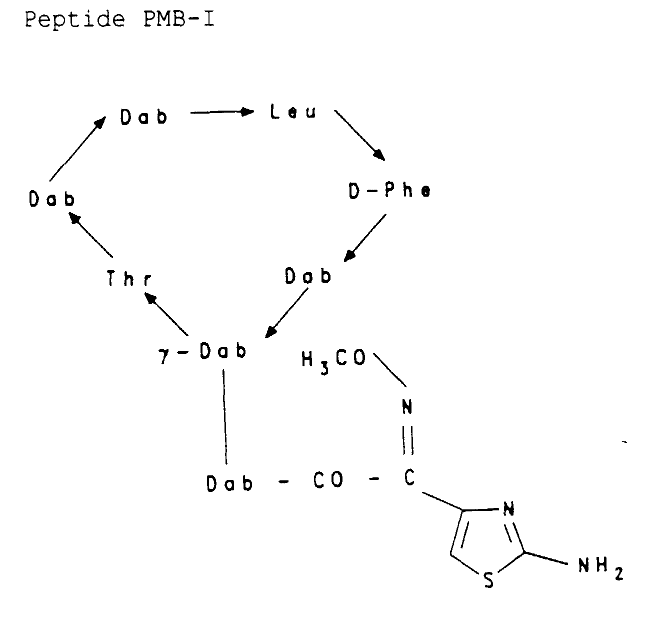 ou ses sels physiologiquement compatibles.

3. Composé de Formule 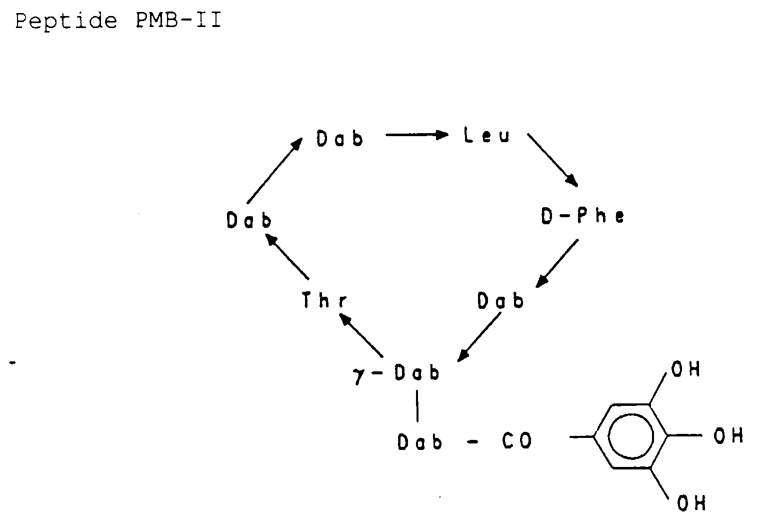 ou ses sels physiologiquement compatibles.

4. Composé de Formule 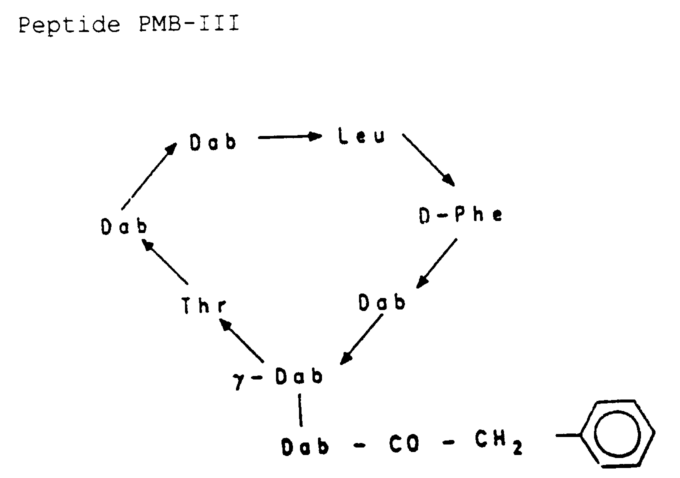 ou ses sels physiologiquement compatibles.

5. Utilisation d'au moins un composé selon une ou plusieurs des revendications 1 à 4, pour la fabrication d'une préparation pharmaceutique destinée à l'amélioration de la résorption de peptides et de protéines à travers la muqueuse.

6. Préparation pharmaceutique, comprenant
a) une quantité pharmacologiquement active d'un, de deux ou de trois peptides ou protéines, contenant respectivement de 3 à 225 aminoacides, en particulier de 3 à 51 aminoacides, ou leurs sels physiologiquement compatibles et
b) au moins un auxiliaire de Formule générale I. 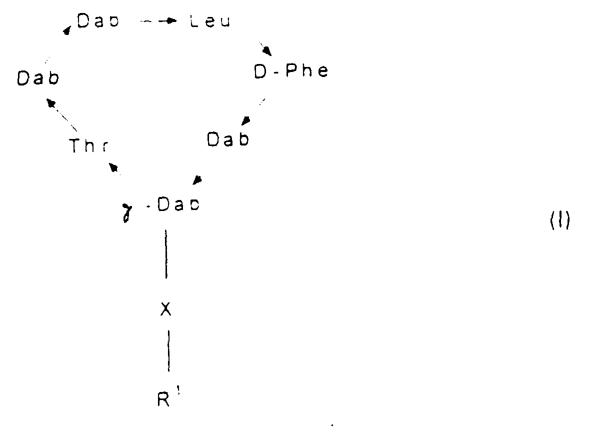
ou un sel physiologiquement compatible de cet auxiliaire,
Formule I dans laquelle
X représente la lysine, l'ornithine, l'acide 2,4-diaminobutyrique ou l'arginine,
R¹ représente un radical acyle de Formule II lié à un azote 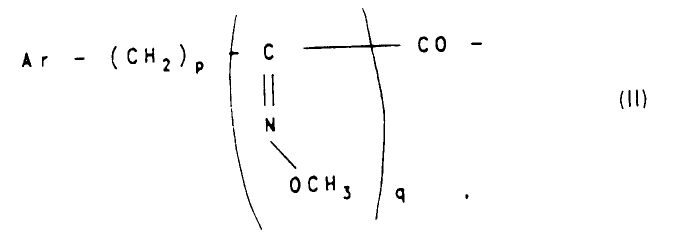
formule dans laquelle Ar représente un radical phényle éventuellement substitué par 1, 2 ou 3 groupes identiques ou différents choisis parmi les groupes hydroxy, alcoxy en C₁-C₄, amino, carboxy, alkylamino en C₁-C₄ et les atomes d'halogène, ou
un radical 2-aminothiazol-4-yle,
p signifie un nombre entier compris entre zéro et 4,
q zéro ou 1,
et les aminoacides peuvent être respectivement sous la forme D ou L.

7. Préparation pharmaceutique selon la revendication 6, caractérisée en ce qu'elle contient un peptide ou une protéine contenant de 3 à 225 aminoacides.

8. Préparation pharmaceutique selon une ou plusieurs des revendications 6 et 7, caractérisée en ce qu'elle contient un peptide ou une protéine contenant de 3 à 51 aminoacides.

9. Procédé de fabrication d'une préparation pharmaceutique selon une ou plusieurs des revendications 6 et 7, caractérisée en ce que, l'on met
a) un, deux ou trois peptides ou protéines, contenant respectivement de 3 à 225 aminoacides, en particulier
de 3 à 51 aminoacides, ou leurs sels physiologiquement compatibles et
b) au moins un auxiliaire de Formule générale I
sous une forme de présentation appropriée pour l'application sur la muqueuse.
